# EUROPEAN PATENT APPLICATION

(11) **EP 3 460 454 A1**
(43) Date of publication of application: **27.03.2019**
(21) Application number: 17799583.4
(22) Date of filing: 28.04.2017
(51) Int. Cl.: G01N 21/552, G01N 21/03, G01N 21/25

(54) **BIOSENSOR AND METHOD FOR ANALYZING SPECIMEN BY USING SAME**

(30) Priority: 17.05.2016 KR 20160060161
(71) Applicant: Plexense, Inc., Giheung-gu, Yongin-si Gyeonggi-do 17015 (KR)
(72) Inventor: KIM, Gibum, Daejeon 34329 (KR); KIM, Taeyoung, Seongnam-si Gyeonggi-do 13569 (KR); KIM, Chealkon, Busan 47179 (KR); HWANG, Hyejin, Yongin-si Gyeonggi-do 16861 (KR); LEE, Jiyoung, Gyeryong-si Chungcheongnam-do 32837 (KR)
(74) Representative: Vossius & Partner Patentanwälte Rechtsanwälte mbB
(86) International application number: PCT/KR2017/004546
(87) International publication number: WO 2017/200225

(57) **Abstract**

The present invention relates to a biosensor. The biosensor according to an embodiment of the present invention comprises: a substrate(11) having a predetermined length; a sensing unit(10) having a thin film layer(13) formed by dispersing and arranging conductive nanoparticles or nanostructures(14) on at least one of the both sides of the substrate(11) to cause an LSPR phenomenon and being immersed in a target sample(3) to bind a target analyte in the target sample(3) to the thin film layer(13); and a gripping unit(20) connected to one end of the substrate(11) and gripped by a user.

## Description

### Technological Field

The present invention relates to a biosensor and a sample analysis method using the biosensor.

### Background Art

An assay based on localized surface plasmon resonance (LSPR) is a method in which a sample concentration-dependent refractive index change is analyzed by measuring a change in the intensity or wavelength of reflected or transmitted light through a metal thin film layer, formed by coating metal nanoparticles on a transparent substrate surface, while irradiating the thin film layer with light. Analytical methods utilizing such an LSPR phenomenon for assaying biological or non-biological samples have been investigated to overcome disadvantages, such as complex sample processing and long analysis time, of existing fluorescence-based analysis methods.

Common methods for analyzing biological samples such as nucleic acids and proteins can be divided into two major areas as follows. The first one is a method of analyzing the concentration of a sample by measuring optical absorbance using an ultraviolet-visible spectroscopic method. In the method, an absorbance is measured by passing light of a certain intensity through a sample and then comparing intensity of light before and after the passage. Such an optical absorbance measurement method measures only the concentration of a specific functional group contained in the sample. Therefore, there exists inconvenience of applying an additional analytical method or more in order to quantitatively analyze the reactivity and activity of a specific binding substance in a biological reaction. Furthermore, the method offers a low analytical sensitivity of 10⁻⁶M and thus it is not suitable for analyzing a biological sample that typically requires a high analytical sensitivity of 10⁻¹²M.

The second one is to utilize enzyme immunoassay, as disclosed in the patent documents of the following prior art documents. Enzyme immunoassay is a method commonly used for quantitatively analyzing the reactivity and activity of a specific sample at a high analytical sensitivity of 10⁻¹²M. The enzyme immunoassay uses a quantitative analysis method in which a sample is analyzed using an enzyme-labeled antibody formed by chemical binding of an enzyme such as peroxidase or galactosidase with an antibody in a target-specific antigen-antibody reaction. Alternatively, fluorescence immunoassay can be used in which a sample is analyzed using an antigen or antibody labeled with a fluorescent dye such as fluorescein and rhodamine and a fluorescence analyzer.

These analytical methods are widely used because they permit to analyze, with an excellent detection sensitivity, the reactivity and activity of the reaction between a reactant and a target analyte in a sample. However, they still have problems of a long assay time and high assay cost because of complicated sample processing, labeling of a sample or target analyte with a fluorescent dye, or use of an expensive analyzer. In particular, enzyme immunoassay or fluorescence immunoassay has difficulties in rapid screening of a large number of libraries during drug development or biomarker development due to a long assay time and necessity of using a separate target-specific antibody depending on the target analyte.

Therefore, there is a desperate need for a solution to the problem of the conventional sample analysis method.

### Detailed Description of the Invention

### Problems to be Solved by the Invention

The present invention is intended to solve the aforementioned problems of the conventional arts. One aspect of the present invention is to form a thin film layer in which conductive nanoparticles or nanostructures are dispersedly disposed on at least one of one surface and the other surface of the substrate and to provide a biosensor that detects a sample by inducing an LSPR phenomenon.

A different aspect of the present invention is to provide a biosensor that has a narrow portion having a relatively narrow width at a predetermined position of a substrate so as to prevent a sample from rising along a gap between two immediate substrates or between a substrate and an inner wall of a cuvette.

Another aspect of the present invention is to provide a relatively simple and inexpensive sample analysis method using a biosensor utilizing an LSPR phenomenon without a separate sample pretreatment process.

### Means for Solving the Problems

A biosensor according to an embodiment of the present invention comprises a substrate having a predetermined length and a thin film layer, formed by dispersing and arranging conductive nanoparticles or nanostructures on at least one of the both sides of the substrate to cause localized surface plasmon resonance (LSPR) phenomenon, which is immersed in a target sample to bind a target analyte in the target sample; and a gripping unit connected to one end of the substrate and gripped by a user.

In addition, a biosensor according to an embodiment of the present invention further comprises a cap connected to the substrate and the gripping unit and releasably inserted into a cuvette accommodating the target sample.

In addition, a biosensor according to an embodiment of the present invention further comprises a fixing unit which is arranged on an outer surface of the cap and has deformable resilience capable of being close contact with an inner circumferential surface of the cuvette when the cap is inserted into the cuvette.

In addition, in a biosensor according to an embodiment of the present invention, the fixing unit is formed to be extended and bent from an outer surface of the cap.

In addition, in a biosensor according to an embodiment of the present invention, an outer surface portion of the cap facing the fixing unit is recessed.

In addition, in a biosensor according to an embodiment of the present invention the substrate comprises a narrow portion having a relatively narrow width at a predetermined height with respect to the other end of the substrate.

In addition, in a biosensor according to an embodiment of the present invention, the narrow portion is formed as an ascend prevention groove dented concavely from the side of the substrate.

In addition, in a biosensor according to an embodiment of the present invention, the ascend prevention grooves are formed on both sides of the substrate.

In addition, in a biosensor according to an embodiment of the present invention, a plurality of the ascend prevention grooves are formed along the sides and spaced apart in the lengthwise direction of the substrate.

In addition, in a biosensor according to an embodiment of the present invention, a plurality of the sensing units are spaced apart from one another and placed side by side.

In addition, a biosensor according to an embodiment of the present invention further comprises a pair of guards, disposed opposite to each other with the sensing unit therebetween, to protect the sensing part.

In addition, a biosensor according to an embodiment of the present invention comprises an adaptor, disposed under bottom surface of a cuvette accommodating the target sample thereinto, to adjust a height of the cuvette.

In addition, in a biosensor according to an embodiment of the present invention, the adaptor is formed in a block shape, and comprises an engaging groove on the outer surface of the adaptor for binding to a bottom surface of the cuvette.

In addition, in a biosensor according to an embodiment of the present invention, the sensing unit is immersed in the target sample, and then the target sample is analyzed by irradiating the cuvette with light.

In addition, in a biosensor according to an embodiment of the present invention the analysis of the target sample is protein assay, immunoassay, kinetic analysis, or small molecule detection.

In another aspect, a sample analysis method using a biosensor according to an embodiment of the present invention comprises (a) preparing the biosensor according to any one of the claims 1 to 15; (b) immersing a sensing unit of the biosensor in a detection sample containing a detection substance and immobilizing the detection substance; and (c) immersing the sensing unit in a target analyte by inserting the sensing unit on which the detection substance is immobilized in a cuvette accommodating the target analyte which specifically binds to the detection substance.

In addition, a sample analysis method using a biosensor according to an embodiment of the present invention may further comprise a step of immersing the sensing unit of the biosensor in a rinsing solution between the steps (b) and (c).

In addition, a sample analysis method using a biosensor according to an embodiment of the present invention may further comprise a step of measuring absorbance, after the (c), by arranging the biosensor of which the sensing unit is inserted in the cuvette in a spectroscopic analyzer.

In addition, in a sample analysis method using a biosensor according to an embodiment of the present invention the sensing unit is inserted and immersed in a cuvette containing the detection substance in the (c), and may further comprise a step of measuring absorbance by placing the biosensor of which the sensing unit is immersed in the cuvette containing the detection substance into a spectroscopic analyzer between the steps (b) and (c).

In addition, in a sample analysis method using a biosensor according to an embodiment of the present invention may further comprise a step of measuring absorbance by placing the biosensor of which the sensing unit is inserted and immersed in a cuvette containing a rinsing solution into a spectroscopic analyzer.

The features and advantages of the present invention will become more apparent from the following description with reference to the appended drawings.

Prior to this, it should be understood that the terms and words used in the present specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

### Effects of the Invention

A biosensor of the present invention quantitatively detects a sample by inducing an LSPR phenomenon on the thin film layer of metal nanoparticles or nanostructures dispersedly disposed on at least one of one surface and the other surface of the substrate. The biosensor can easily induce the reaction between biological samples or between biological and non-biological samples, without a separate sample pretreatment process.

In addition, in a biosensor of the present invention, the sensing unit, immersed in a cuvette containing a sample, for analyzing the sample has a narrow portion having a relatively narrow width formed at a predetermined position of the substrate constituting the sensing unit. The narrow portion offers an advantage of preventing the sample from rising by a capillary force along a gap between two immediately adjacent substrates in parallel or between the substrate and the inner surface of the cuvette.

In addition, a sample analysis method using a biosensor of the present invention is based on a LSPR phenomenon and thus does not necessitate chromophore labeling, unlike enzyme immunoassay that requires a complicated step of labeling a sample molecule with a fluorescent dye. Therefore, the biosensor permits to quantitatively analyze a sample through a simple detection process only with a visible light spectroscopic analyzer.

### Brief Description of the Drawings

Fig. 1 is a perspective view of a biosensor according to an embodiment of the present invention.
Fig. 2a and 2b are cross-sectional views taken along the line A-A' of Fig. 1.
Fig. 3 is a side view of a biosensor which is inserted into a cuvette, according to an embodiment of the present invention.
Fig. 4 is a front view of a biosensor which is inserted in a cuvette, according to an embodiment of the present invention.
Fig. 5 is a perspective view of a biosensor according to another embodiment of the present invention.
Fig. 6 is a perspective view of an adaptor according to an embodiment of the present invention.
Fig. 7 is a graph showing a signal of a biosensor according to an embodiment of the present invention.
Fig. 8 is a flowchart illustrating a method of analyzing a sample using a biosensor according to an embodiment of the present invention.
Fig. 9 is a graph of absorbance change analyzed by a sample analysis method using a biosensor according to an embodiment of the present invention.

### Best Mode for Carrying out the Invention

The objectives, specific advantages, and novel features of the present invention will become more apparent from the following detailed description and preferred embodiments with reference to the appended drawings. It should be noted that the same reference numerals are denoted to the elements of the drawings in the present specification with the same numerals as possible, even if they are displayed in other drawings. Also, the terms "the first", "the second" and the like are used to distinguish one element from another and thus the element is not limited thereto. Hereinafter, in the description of the present invention, a detailed explanation of related known arts which may unnecessarily obscure the gist of the present invention will be omitted.

Hereinafter, preferred embodiments of the present invention will be described in detail with reference to the appended drawings.

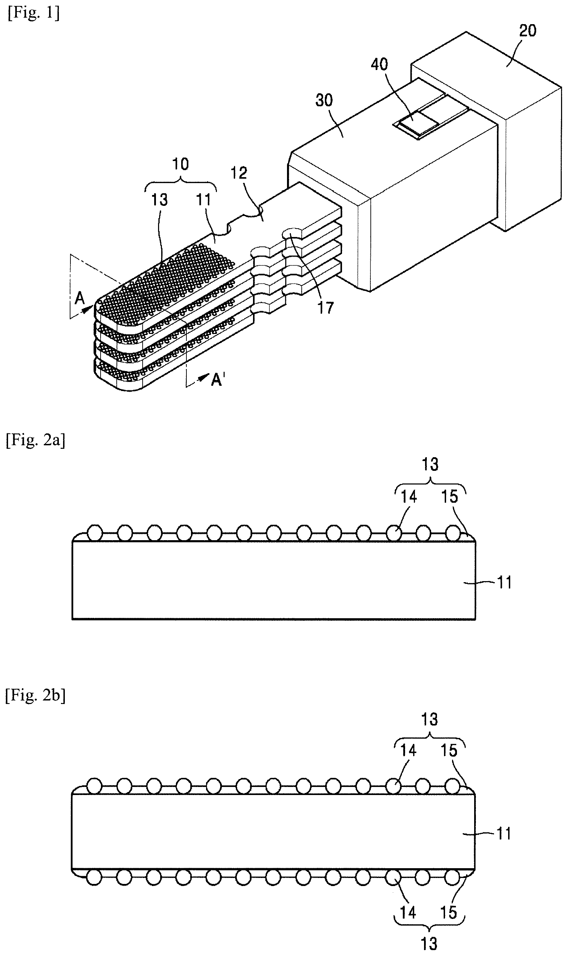

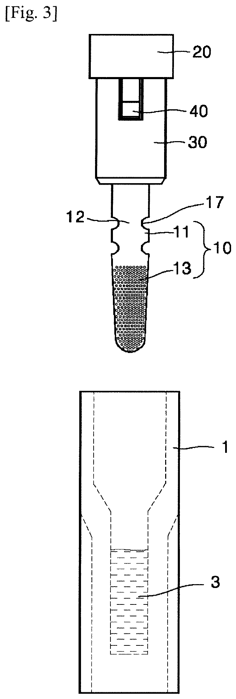

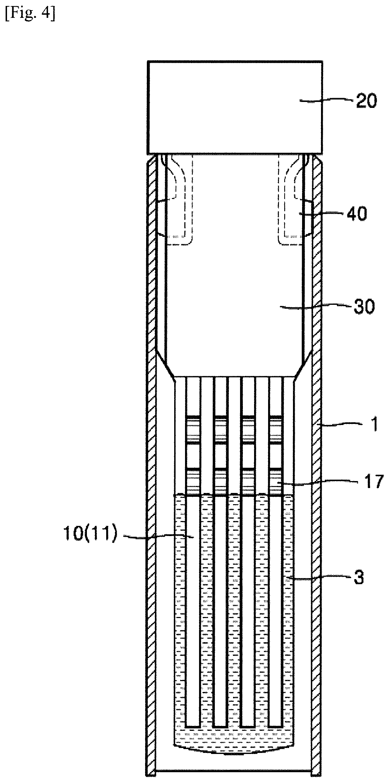

Fig. 1 is a perspective view of a biosensor according to an embodiment of the present invention; Fig. 2 is cross-sectional views taken along the line A-A' of Fig. 1; Fig. 3 is a side view of a biosensor inserted into a cuvette according to an embodiment of the present invention; Fig. 4. is a front view of a biosensor inserted into a cuvette according to an embodiment of the present invention.

As illustrated in Fig. 1 to Fig. 4, a biosensor according to an embodiment of the present invention comprises a substrate(11) having a predetermined length; a sensing unit(10) having a thin film layer(13) formed by dispersedly disposing conductive nanoparticles or nanostructures(14) on at least one of the both surfaces of the substrate(11) to induce an LSPR phenomenon and being immersed in a target sample(3) to induce a reaction between a target analyte in the target sample(3) and a surface of the thin film layer(13); and a gripping unit (20) connected to one end of the substrate(11) and gripped by a user.

### Mode for Carrying out the Invention

A biosensor according to an embodiment of the present invention relates to a sensor for detecting a sample utilizing an LSPR phenomenon, and comprises a sensing unit(10) and a gripping unit (20).

Surface plasmon resonance (SPR) refers to a phenomenon of the propagation of surface plasmon polaritons which are generated on or near the surface of conductive materials by coupling of electrons and photons having a specific wavelength. In general, SPR is a phenomenon of the collective oscillation of conduction band electrons propagating along the interface between a metal with a negative dielectric constant and a medium with a positive dielectric constant. SPR results in enhanced intensity in comparison with an incident electromagnetic wave and shows characteristics of an evanescentwave which exponentially decays as getting far-off perpendicularly from the interface.

SPR can be classified as a propagating surface plasmon resonance (PSPR) observed at the interface between a dielectric material and a 10-200 nm-thick flat metal surface; and localized surface plasmon resonance (LSPR) observed from nanoparticles or nanostructures. A biosensor based on LSPR detects a change in the LSPR wavelength showing a maximum absorption or scattering which depends on a change of the chemical and physical environment on the surface (for example, a change in refractive index of a medium near the surface) of the nanoparticles or nanostructure. The detection of the LSPR wavelength change permits to distinguish specific molecules or to analyze concentration of specific molecules in a medium; LSPR is highly sensitive to the change of refractive index and that allows label-free detection. A biosensor according to the present invention is fabricated such that LSPR is applied.

At this time, LSPR occurs in the sensing part(10) which comprises the substrate(11) and the thin film layer(13).

Here, the substrate(11) is a plate-shaped part having a predetermined length. The substrate(11) is not necessarily limited to a flat plate, but may be formed in various shapes such as a curved shape or a convexo-concave( 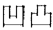 ) shape. However, hereinafter, the description assumes a flat plate.

On the other hand, the substrate(11) may be an optically transparent or opaque substrate(11), but the optically transparent substrate(11) is preferable. The optically transparent substrate(11) may be made of, for example, glass or a polymer material having a certain degree of optical transparency. The polymer material may comprise polycarbonate(PC), polyethylene terephthalate(PET), polymethyl methacrylate(PMMA), triacetyl cellulose(TAC), cycloolefin, polyarylate, polyacrylate, polyethylene naphthalate, polybutylene terephthalate or polyamide. However, the polymer material is not necessarily limited thereto. An opaque substrate(11) may be made of sapphire, silicon single crystal. However, the material of the substrate(11) is not limited to the aforementioned materials and various other materials can be utilized in consideration of the conditions of the target analyte, the fabrication process, and the like.

The thin film layer(13) is a layer formed on at least one of the both surfaces of the substrate(11) and is formed by dispersedly disposing conductive nanoparticles or nanostructures(14) that cause LSPR. The thin film layer(13) may be formed on only one surface (see Fig. 2a) of the substrate(11) or on both surfaces(see Fig. 2b) of the substrate(11). On the other hand, the conductive nanoparticles or nanostructures(14) may have any shape selected from a nanosphere, a nanotube, a nanocolumn, a nanorod, a nanopore, a nanowire, or combinations thereof. The nanoparticles or nanostructures may be completely filled, porous or hollowed depending on the shape. The conductive nanoparticles or nanostructures(14) may be conductive particles of carbon, graphite, metalloid, metal, metalloid alloy, metal alloy, conductive metal oxide, conductive metal nitride; or core-shell structure particles in which a conductive layer such as a metal thin film is coated on an insulating core. However, the conductive nanoparticles or nanostructures(14) are not necessarily limited to the aforementioned shapes and materials.

On the other hand, the conductive nanoparticles or nanostructures(14) are immobilized (see Fig. 2) on the substrate(11) by a binder(15) wherein the binder(15) may be an ionic polymer such as poly diallydimethylammonium chloride, poly allylamine hydrochloride, poly 4-vinylbenzyltrimethyl ammonium chloride, polyethyleneimine, poly acrylic acid, poly sodium 4-styrene sulfonate, poly vinylsulfonic acid, poly sodium salt, poly amino acids or a mixture thereof. However, the binder(15) is not limited to the aforementioned polymer, as long as it is a material capable of immobilizing nanoparticles or nanostructures(14) on the substrate(11).

The sensing unit(10) formed by disposing the thin film layer(13) on the substrate(11) is immersed in the target sample(3). At this time, the thin film layer(13) is also immersed in the target sample(3) as the substrate(11) is immersed from its free end in the target sample(3). Here, the free end of the substrate(11) refers to the opposite end of one end of the substrate(11) connected to the gripping unit (20) to be described later. When the thin film layer(13) is immersed in the target sample(3), a target analyte in the target sample(3) binds to the thin film layer(13).

At this time, a detection substance that specifically binds with the target analyte may be immobilized on the thin film layer(13) in order for the thin film layer(13) to bind with the target analyte. The detection substance may be, for example, a low molecular weight compound, an antigen, an antibody, a protein, a peptide, a DNA, an RNA, a PNA, an enzyme, an enzyme substrate, a hormone receptor, and a synthetic reagent having a functional group. However, the aforementioned detection substances are just examplary ones and thus the detection substance is not necessarily limited thereto. The detection substance may comprise any known substances, including combinations of such substances, that combine with the target analyte. The detection substance is immobilized on the thin film layer(13), i.e., conductive nanoparticles or nanostructures(14), or on the binder (15); and specifically binds to the target analyte, thereby binding the target analyte to the thin film layer(13). However, the detection substance is not necessarily immobilized on the thin layer(13).

On the other hand, the sensing unit(10) may be one or more. When the sensing units(10) are plural, they are spaced apart from each other by a predetermined distance and arranged side by side. In this case, the sensing units(10) are arranged such that one surface of the substrate(11) is opposed to one surface or the other surface of another substrate(11). One or more of the sensing units(10) are connected to and fixed to the gripping unit (20).

Here, the gripping unit (20) is a unit held by a user and is connected to one end of the substrate(11). Thus, the user can hold the gripping unit (20) and immerse the sensing unit(10) into the cuvette(1) in which the target sample(3) is accommodated. Here, the cuvette(1) is a container to accommodate the target sample(3) for the spectroscopic analysis of a target sample(3). However, the target sample(3) is not necessarily accommodated in the cuvette(1) when the sensing unit(10) is immersed. Nevertheless, in the general sample analysis process, the target sample(3) is prepared in the cuvette(1) and the spectroscopic analysis is performed in a state in which the sensing unit(10) is immersed in the cuvette(1). Therefore, hereinafter, it is assumed that the target sample(3) is accommodated in the cuvette(1).

According to the aforementioned descriptions, the biosensor according to the present embodiment has a structure in which the thin film layer(13) having the conductive nanoparticles or nanostructures(14) dispersedly disposed on one surface of the substrate(11) is formed. The thin film layer(13) causes LSPR which can be utilized for identifying the specific target analyte and determining the concentration of the target analyte in a medium. The biosensor allows a label-free detection of the target analyte.

Meanwhile, a biosensor according to the present embodiment may further comprise a cap(30). Here, the cap(30) is configured to be removably inserted into the cuvette(1) and to close or seal the open inlet of the cuvette(1). The cap(30) is disposed under the gripping unit (20) and connects the gripping unit (20) and the substrate(11). The cap(30) is held in contact with the inner surface of the cuvette(1) and fix the substrate(11) so that the sensing unit(10) does not move in the cuvette(1).

Here, the sizes of the cuvette(1) are not all uniform. Therefore, depending on the size of the cuvette(1), a gap may be created between the outer surface of the cap(30) and the inner surface of the cuvette(1). Due to the gap, the cap(30) remains unfixed to the cuvette(1), making it difficult to accurately analyze the target sample(3). To avoid this problem, the biosensor may further comprise a fixing unit (40) to fix or secure the sensing unit(10) regardless of the size of the cuvette(1).

The fixing unit (40) is arranged on the outer surface of the cap (30). With this arrangement, the original position or shape of the fixing unit (40) is changed to create resilience when the cap(30) is inserted into the cuvette(1). The fixing unit (40) is brought into close contact with the inner circumferential surface of the cuvette(1) by the resilience. The sensing unit(10) connected to the cap(30) is fixed or secured in the cuvette(1) when the fixing unit (40) disposed on the cap(30) is in close contact with the cuvette(1).

Specifically, the fixing unit (40) may be an elastic unit that is deformed while being pressed by the inner surface of the cuvette(1) and comes into close contact with the inner surface of the cuvette(1) by elasticity when the cap(30) is inserted into the cuvette(1). The fixing unit (40) may use the inherent elasticity of the elastic material such as rubber or the like, or may use the properties of a unit such as a spring. However, the fixing unit (40) does not necessarily have to use the elasticity of the elastic material or the unit, but can be implemented through a predetermined structure, which will be described in detail below (see Fig. 4).

The fixing unit (40) may extend from the outer surface of the cap(30) and may be bent in a predetermined direction. For example, the fixing unit(40) may extend outward from the outer surface of the cap(30) and may be bent in parallel to the outer surface of the cap(30) to form an inverted L shape. The outwardly protruding protrusion formed at one end of the fixing unit (40) is pressurized against the inner surface of the cuvette(1), and as a result, the fixing unit (40) can be brought into close contact with the inner surface of the cuvette(1) by tension. At this time, since the fixing unit (40) is moved toward the cap(30) when pressured, a portion of the outer surface of the cap(30) opposite to the fixing unit(40) may be recessed.

Alternatively, the fixing unit(40) may extend from the inner surface of the recessed portion of the cap(30) and the protrusion may be formed outward from the outer surface of the cap(30) to have an "L" shape.

Consequently, the fixing unit(40) may extend from the outer surface of the cap(30) and be freely modified into various structures so long as it can be brought into close contact with the inner surface of the cuvette(1) by tension when the cap(30) is inserted into the cuvette (1).

Meanwhile, in a biosensor according to the present embodiment, the substrate(11) may have a narrow portion(12). Here, the narrow portion(12) is a portion where the width of the substrate(11) is relatively narrowed at a predetermined height with respect to the other end (free end) of the substrate(11), as compared with other portions.

In order to analyze the target sample(3), the cuvette(1) is irradiated with light from the outside of the cuvette(1) in a state in which the sensing unit(10) is inserted. When the sensing unit(10) is inserted into the cuvette(1), a capillary force is created in the gap between the sensing unit(10) and the inner surface of the cuvette(1) or in the gap between the immediately arranged sensing units(10) in parallel and that leads to a rise of the target sample(3) (see Fig. 4). Due to the rise of the target sample(3), the target sample(3) moves to a position beyond the region of the substrate(11) on which the thin film layer(13) disposed, so that the amount of the target sample(3) required for the analysis is increased and the reliability of the analysis is seriously degraded. The narrow portion(12) is contrived to solve this problem. The target sample(3) rises along the substrate(11) by the attractive force between the target sample(3) and the substrate(11). Thus, when the width of the substrate(11) becomes narrowed in the narrow portion(12), the area of contact between the substrate(11) and the target sample(3) becomes small, so that the target sample(3) no longer rises.

Specifically, the narrow portion(12) can be formed through the formation of the ascend prevention groove(17) recessed from the side surface of the substrate(11) concavely. Since the ascend prevention groove(17) is recessed by a predetermined depth from one side surface to the other side surface of the substrate(11), the width of the substrate(11), *i.e.* the distance between the both side surfaces of the substrate(11), at the position where the ascend prevention groove(17) is formed is small. The ascend prevention groove(17) may be formed only on one side surface of the substrate(11), but may be formed on both side surfaces of the substrate(11). In the case where the ascend prevention grooves(17) are formed on the both side surfaces of the substrate(11), they may be formed so as to face each other, but are not necessarily limited thereto. The ascend prevention grooves(17) may be staggered in a zigzag pattern. The ascend prevention grooves(17) may be formed in plurality along the side surfaces of the substrate(11) at a predetermined distance in the lengthwise direction.

The ascend prevention groove(17) may be formed to be rounded in shape but it is not necessarily formed in such a shape. The ascend prevention groove(17) may be recessed in any shape so long as the width of the substrate(11) is narrowed. At this time, since there is no particular limitation on the width of the ascend prevention groove(17), *i.e.* the distance perpendicular to the depth of the ascend prevention groove(17), the width of the ascend prevention groove(17) may range from a predetermined height of the substrate(11) to one end thereof.

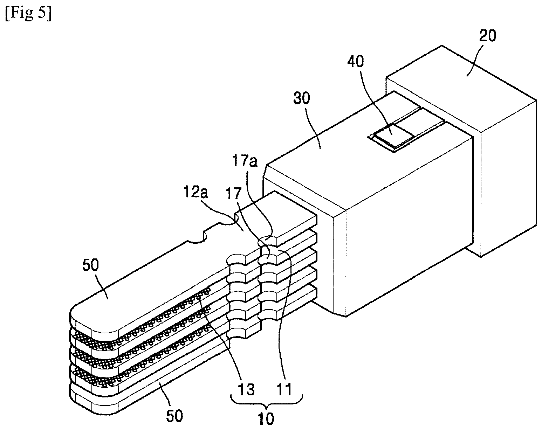

Fig. 5 is a perspective view of a biosensor according to another embodiment of the present invention. As illustrated in Fig. 5, the biosensor according to another embodiment of the present invention may further comprise a pair of guards(50) to protect sensing unit(10). Here, the pair of guards(50) are units that are disposed opposite to each other with the sensing unit(10) therebetween, spaced apart from the sensing unit(10). A pair of the guards(50) is arranged so that a plurality of sensing units(10) are interposed between the pair of guards(50) even when a plurality of sensing units(10) are arranged. Since the guards(50) are disposed on each of the outermost sides of the sensing unit(10), the sensing unit(10) is prevented from touching the inner surface of the cuvette(1) and is protected from external impact and the like. The guard(50) may be formed in the same shape as that of the substrate(11), but the shape of the guard(50) is not necessarily limited thereto. When the guard(50) is formed in a plate shape, the guard(50) may also be formed with a narrow portion(12a) whose width is relatively narrow at a predetermined height, because the sample(3) may rise in the gap between the guard(50) and the sensing unit(10) arranged in parallel or between the guard(50) and the inner surface of the cuvette(1). At this time, the narrow portion(12a) may be formed by recessing the ascend prevention groove(17a) on the side surface of the guard(50). However, it is not necessarily required to form the narrow portion(12a) on the guard(50) or to form the narrow portion(12a) by the ascend prevention groove(17a). In addition, since light is irradiated to the sensing unit(10) during the sample analysis process, the guard(50) is preferably formed to be transparent, but the guard(50) is not necessarily required to be transparent.

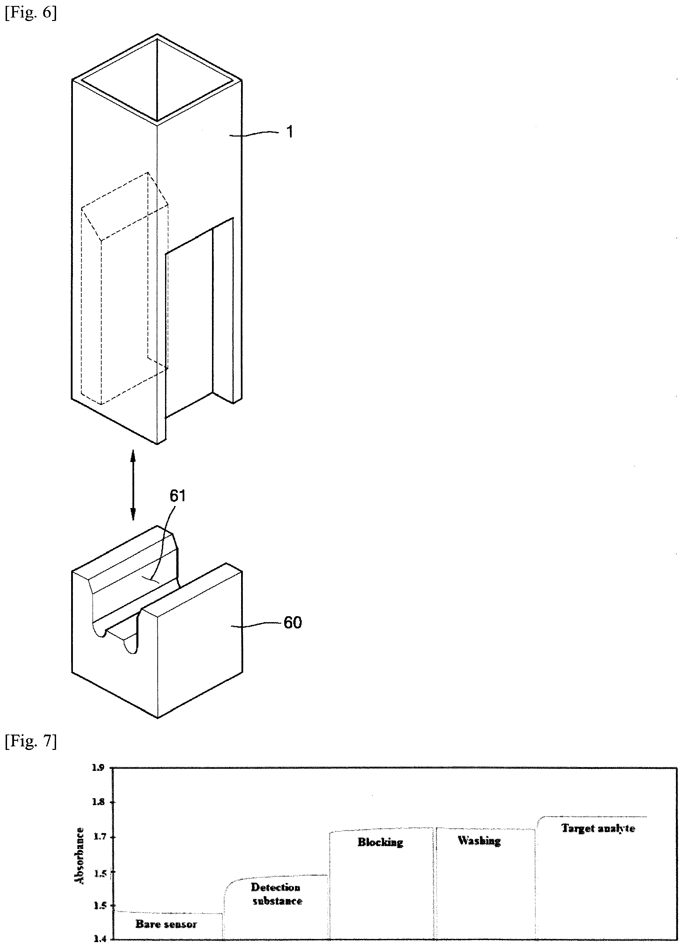

Fig. 6 is a perspective view of an adaptor(60) for a biosensor according to an embodiment of the present invention. As illustrated in Fig. 6, the biosensor according to the present embodiment may further comprise an adaptor(60). Here, the adaptor(60) is a part to adjust the height of the cuvette(1). In order to analyze the target sample(3), the sensing unit(10) is inserted into the cuvette(1) and then light is irradiated from the outside of the cuvette(1) toward the thin film layer(13) of the sensing unit(10) (see FIG. 3). In general, since the height, width, and focus of the light are different depending on the equipment used for light irradiation, the manufacturer of the cuvette(1) designs the height of the cuvette(1) in accordance with the predetermined light irradiation equipment. Therefore, when the cuvette(1) which is not matched with the light irradiation equipment is used, the cuvette(1) cannot be irradiated with light at the proper height required for the sample analysis, limiting versatility of the cuvette(1). The height of the cuvette(1) can be adjusted by arranging the adaptor(60) under the bottom surface of the cuvette(1) in the case where the use of the cuvette(1) is problematic because the light irradiation equipment does not match the cuvette(1). Therefore, the position of light irradiation can be conveniently adjusted by controlling the height of the cuvette(1), irrespective of the type of the light irradiation equipment and the cuvette(1).

Specifically, the adaptor(60) is formed in a block shape having a predetermined height and has an engaging groove(61) recessed or perforated from the outer surface. The adaptor(60) and the cuvette(1) are combined by inserting the bottom surface of the cuvette(1) into the engaging groove(61). Here, the adaptor(60) can be fabricated to have a different height and combined with the cuvette(1) to adjust the height of the cuvette(1).

The biosensor according to the present embodiment is configured to analyze target sample(3) by irradiating the cuvette(1) with light in a state in which the sensing unit(10) is immersed in the target sample(3), i.e. in a state where the sensing unit(10) is inserted into the cuvette(1). At this time, the analysis of the target sample(3) comprises protein assay, immunoassay, kinetic analysis, and small molecule detection. Conventionally the protein assay, immunoassay, kinetic analysis, and small molecule detection were performed separately using a separate device. However, the biosensor according to the present embodiment makes it possible to perform all the aforementioned analyses.

Fig. 7 is a graph showing a signal change measured using a biosensor according to an embodiment of the present invention. Fig. 7 shows a series of signal changes continuously measured under various conditions using the biosensor according to the present invention. In the first step, the background signal of the biosensor according to the present invention was measured in PBS buffer solution. In the second step, the biosensor according to the present invention was inserted into the cuvette containing an antibody (detection substance) to measure the signal for antibody immobilization. In the third step, the signal was measured after treating the empty portion (where the detection substance was not immobilized with the antibody) of the metal nanoparticle thin film layer surface with a blocking substance. In the fourth step, extra molecules of the blocking substance not involved in the blocking were removed by washing with a rinsing solution and a signal was measured. In the fifth step, the biosensor according to the present invention was inserted into a cuvette containing an antigen (target analyte) to induce a reaction between the antigen and the immobilized antibody, and the increase of the signal was measured. These observations confirmed a signal change in each step. It was further confirmed that a signal appeared even when the signal was measured after immersing the biosensor according to the present invention into the rinsing solution between the second and third steps (not shown here). From these results, it can be seen that the biosensor according to the present invention operates showing a specific signal value depending on the substance to be detected.

Hereinafter, a sample analysis method using the biosensor according to the present invention will be described.

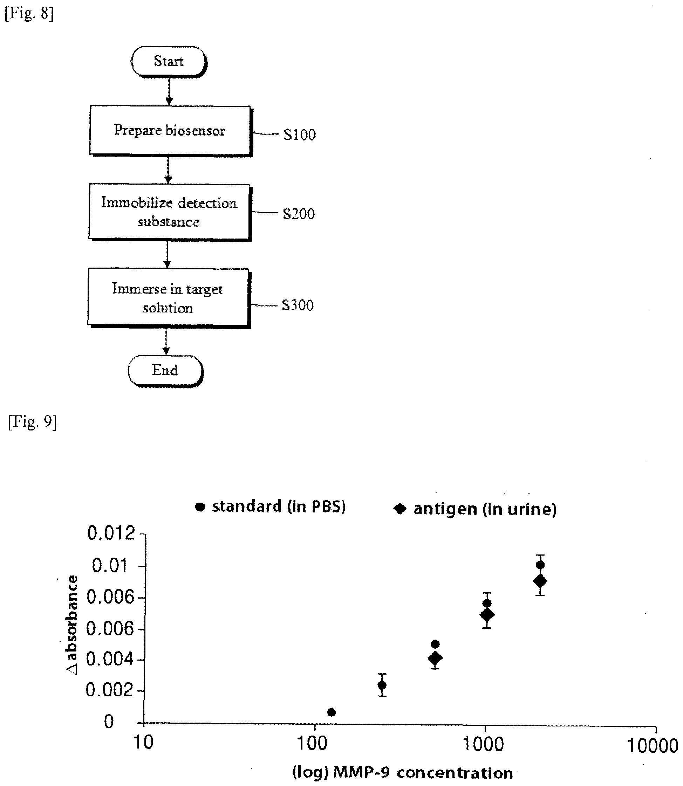

Fig. 8 is a flowchart illustrating a sample analysis method using a biosensor according to an embodiment of the present invention, and Fig. 9 is a graph of absorbance change analyzed by the sample analysis method using a biosensor according to an embodiment of the present invention.

As illustrated in Fig. 8, the sample analysis method using a biosensor according to an embodiment of the present invention comprises: (a) a step of preparing a biosensor according to any one of the above embodiments (S100); (b) a step of immobilizing a detection substance by immersing the sensing unit of the biosensor in a detection sample containing the detection substance (S200); and (c) a step of immersing the sensing unit, immobilized with the detection substance, of the biosensor in the cuvette containing the target analyte specifically binding to the detection substance (S300).

A sample analysis method using the biosensor according to the present embodiment comprises a step of preparing a biosensor (S100), a step of immobilizing a detection substance (S200), and a step of immersing the sensing unit in a target sample (S300). Here, the sample analysis method using the biosensor according to the present embodiment uses the above-described biosensor according to the present invention, so that redundant contents will be omitted or simply described.

According to the sample analysis method using the biosensor according to the present embodiment, any one of the above-described biosensors is prepared (S100), and the sensing unit of the biosensor is immersed in a detection sample containing a detection substance specifically binding to the target analyte to immobilize the detection substance on the thin film layer (S200). At this time, the detection substance may be prepared in a cuvette, and then the sensing unit of the biosensor may be immersed in the cuvette. When the biosensor is inserted into the cuvette and the sensing unit is immobilized with the detection substance, the biosensor inserted in the cuvette can be arranged in a spectroscopic analyzer to measure the absorbance. However, the measurement of absorbance here does not necessarily have to be performed.

When the detection substance is immobilized on the sensing unit, the sensing unit is inserted into the cuvette containing the target analyte solution to immerse the sensing unit in the target analyte (S300). At this time, the detection substance of the sensing unit binds to the target analyte. For example, when the detection substance is an antibody and the target analyte is an antigen, an antigen-antibody reaction is induced.

On the other hand, after the detection substance is immobilized on the sensing unit(S200), the sensing unit of the biosensor can be immersed in the rinsing solution before being immersed in the target analyte solution (S300). The detection substance is immobilized to the sensing unit via, for example, a physical or electrostatic reaction, thereby the immersion of the sensing unit in the rinsing solution removes the bound substance in an unintended manner. At this time, the rinse solution is prepared in a cuvette, and the sensing unit of the biosensor can be inserted into the cuvette to be immersed. When the detection substance is removed, the biosensor inserted in the cuvette can be arranged in a spectroscopic analyzer to measure the absorbance. However, this absorbance measurement is not an essential step to be performed.

After the sensing unit is immersed in the target analyte solution, the target sample can be analyzed by arranging the biosensor in a spectroscopic analyzer while inserting it into the cuvette and then measuring the absorbance. At this time, it is preferable to pre-heat the spectroscopic analyzer before the biosensor is arranged, and to arrange the biosensor in the spectroscopic analyzer as soon as the sensing unit is immersed in the target analyte solution. However, it is not necessary to pre-heat the spectroscopic analyzer in advance.

The results of analyzing the absorbance difference according to the concentration of the enzyme, MMP-9(Matrix Metalloproteinase) in the urine through these steps can be verified in Fig. 9.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the various embodiments disclosed herein are within the scope of the present invention, and the specific scope of the present invention will be additionally defined by the appended claims.

### Industrial Applicability

According to the present invention, it is possible to quantitatively detect a sample by generating an LSPR phenomenon, and to easily induce the reaction between biological samples or between biological and non-biological samples without a separate sample pretreatment process. Therefore, an industrial applicability of the biosensor is recognized.

## Claims

1. A biosensor comprising a sensing unit comprising a substrate having a predetermined length and a thin film layer, formed by dispersing and arranging conductive nanoparticles or nanostructures on at least one of the both sides of the substrate to cause localized surface plasmon resonance (LSPR) phenomenon, which is immersed in a target sample to bind a target analyte in the target sample; and
a gripping unit connected to one end of the substrate and gripped by a user.

2. The biosensor according to claim 1, further comprising a cap connected to the substrate and the gripping unit and releasably inserted into a cuvette accommodating the target sample.

3. The biosensor according to claim 2, further comprising a fixing unit which is arranged on an outer surface of the cap and has deformable resilience capable of being close contact with an inner circumferential surface of the cuvette when the cap is inserted into the cuvette.

4. The biosensor according to claim 3, wherein the fixing unit is formed to be extended and bent from an outer surface of the cap.

5. The biosensor according to claim 4, wherein an outer surface portion of the cap facing the fixing unit is recessed.

6. The biosensor according to claim 1, wherein the substrate comprises a narrow portion having a relatively narrow width at a predetermined height with respect to the other end of the substrate.

7. The biosensor according to claim 6, wherein the narrow portion is formed as an ascend prevention groove dented concavely from the side of the substrate.

8. The biosensor according to claim 7, wherein the ascend prevention grooves are formed on both sides of the substrate.

9. The biosensor according to claim 7, wherein a plurality of the ascend prevention grooves are formed along the sides and spaced apart in the lengthwise direction of the substrate.

10. The biosensor according to claim 1, wherein a plurality of the sensing units are spaced apart from one another and placed side by side.

11. The biosensor according to claim 1, further comprising a pair of guards, disposed opposite to each other with the sensing unit therebetween, to protect the sensing part.

12. The biosensor according to claim 1, further comprising an adaptor, disposed under bottom surface of a cuvette accommodating the target sample thereinto, to adjust a height of the cuvette.

13. The biosensor according to claim 12, wherein the adaptor is formed in a block shape, and comprises an engaging groove on the outer surface of the adaptor for binding to a bottom surface of the cuvette.

14. The biosensor according to claim 1, wherein the target sample is accommodated in a cuvette, the sensing unit is inserted in the cuvette, and the target sample is analyzed by irradiating a light to the cuvette while the sensing unit is immersed in the target sample.

15. The biosensor according to claim 14, wherein the analysis of the target sample is protein assay, immunoassay, kinetic analysis, or small molecule detection.

16. A sample analysis method using a biosensor comprising;
(a) preparing the biosensor according to any one of the claims 1 to 15;
(b) immersing a sensing unit of the biosensor in a detection sample containing a detection substance and immobilizing the detection substance; and
(c) immersing the sensing unit in a target analyte by inserting the sensing unit on which the detection substance is immobilized in a cuvette accommodating the target analyte which specifically binds to the detection substance.

17. The analysis method according to claim 16, further comprising immersing the sensing unit of the biosensor in a rinsing solution between the steps (b) and (c).

18. The analysis method according to claim 16, further comprising measuring absorbance, after the (c), by arranging the biosensor of which the sensing unit is inserted in the cuvette in a spectroscopic analyzer.

19. The analysis method according to claim 16, wherein the sensing unit is inserted and immersed in a cuvette containing the detection substance in the (b), and
further comprising measuring absorbance by placing the biosensor of which the sensing unit is immersed in the cuvette containing the detection substance into a spectroscopic analyzer between the steps (b) and (c).

20. The analysis method according to claim 16, further comprising measuring absorbance by placing the biosensor of which the sensing unit is inserted and immersed in a cuvette containing a rinsing solution into a spectroscopic analyzer.
